(19) **Europäisches Patentamt / European Patent Office / Office européen des brevets**

(11) **EP 1 885 488 B1**

(12) **EUROPEAN PATENT SPECIFICATION**

(45) Date of publication and mention
of the grant of the patent:
**08.07.2015 Bulletin 2015/28**

(51) Int Cl.:
**B01J 20/34** *(2006.01)* **B01D 15/08** *(2006.01)*
**B01D 15/20** *(2006.01)* **C07K 1/22** *(2006.01)*

(21) Application number: **06733425.0**

(22) Date of filing: **22.05.2006**

(86) International application number:
**PCT/SE2006/000599**

(87) International publication number:
**WO 2006/126942 (30.11.2006 Gazette 2006/48)**

(54) **REGENERATION OF A CHROMATOGRAPHY MATRIX**

REGENERATION EINER CHROMATOGRAPHIEMATRIX

REGENERATION D'UNE MATRICE DE CHROMATOGRAPHIE

(84) Designated Contracting States:
**AT BE BG CH CY CZ DE DK EE ES FI FR GB GR HU IE IS IT LI LT LU LV MC NL PL PT RO SE SI SK TR**

(30) Priority: **24.05.2005 US 683952 P**

(43) Date of publication of application:
**13.02.2008 Bulletin 2008/07**

(73) Proprietor: **GE Healthcare Bio-Sciences AB**
**75184 Uppsala (SE)**

(72) Inventors:
• **JOHANSSON, Hans J.**
**S-751 84 Uppsala (SE)**

• **LJUNGLÖF, Anders**
**S-751 84 Uppsala (SE)**
• **ÅBERG, Per-Mikael**
**S-751 84 Uppsala (SE)**

(56) References cited:
**WO-A1-2004/089504   WO-A2-01/27623**
**WO-A2-01/27623   WO-A2-01/32915**
**US-A- 6 080 696   US-B1- 6 972 327**

• **MAJORS R.E.: 'The Cleaning and Regeneration of Reversed-Phase HPLC Columns' LCGC NORTH AMERICA January 2003, pages 19 - 26, XP002293699**

EP 1 885 488 B1

**Description**

Technical field

The present invention relates to chromatography, and more specifically to a process of regenerating chromatography matrices to restore their performance as well as a multi-step process comprising several cycles of regeneration according to the invention.

Background

The term chromatography embraces a family of closely related separation methods based on two mutually immiscible phases brought into contact, wherein one phase is stationary and the other one is mobile. One area wherein chromatography has recently become of great interest is in the biotechnological field, such as for large-scale economic production of novel drugs and diagnostics. Generally, proteins are produced by cell culture, either intracellularly or secreted into the surrounding medium. Since the cell lines used are living organisms, they must be fed with a complex growth medium, containing sugars, amino acids, growth factors, etc. Separation of the desired protein from the mixture of compounds fed to the cells and from other cellular components to a sufficient purity, e.g. for use as a human therapeutic, poses a formidable challenge.

Conventionally, cells and/or cell debris has been removed by filtration. Once a clarified solution containing the protein of interest has been obtained, its separation from the other components of the solution is usually performed using a combination of different chromatographic techniques. These techniques separate mixtures of proteins on the basis of their charge, degree of hydrophobicity, affinity properties, size etc. Several different chromatography matrices are available for each of these techniques, allowing tailoring of the purification scheme to the particular protein involved.

As in all process technology, an important aim is to keep the production costs low. Thus, in order to reduce the number of steps required to obtain a product from a cell culture or lysate, improved chromatographic techniques have been presented. Similarly, chromatography matrices are when possible reused. However, since each use of a chromatography matrix will leave certain traces of the operation just performed, many different cleaning protocols are available for restoring the matrix into its original form. Commonly known materials that need to be removed are e.g. non-eluted proteins and protein aggregates. Another important concern within the pharmaceutical industry is the presence of potentially hazardous materials, such as virus, endotoxins etc, which originates from the cell culture and which need to be removed to avoid cross-contamination between batches.

The most commonly used cleaning is a simple wash with buffer, such as the equilibration buffer. Such washing can only be used to restore the matrix a limited number of times. For a more efficient cleaning, treatments with acid and/or base are frequently used, each removing acid and base-sensitive contaminants respectively. In order to even more efficiently restore the matrix, an alkaline protocol known as Cleaning In Place (CIP) is commonly used with many matrices. The standard CIP involves treatment of the matrix with 1M NaOH, pH 14. Such harsh treatment will efficiently remove undesired fouling such as by protein aggregates and the like, but may on the other hand impair some chromatography matrices. For example, many affinity matrices, wherein the ligands are proteins or proteinaceous, cannot withstand standard CIP, at least not while maintaining their original properties. For example, one of the most commonly used affinity chromatography matrices for purification of antibodies comprises Protein A ligands, but such matrices needs to be cleaned under milder conditions than conventional CIP in order to maintain selectivity and binding capacity. In this context, it is understood that the cleaning is closely related to the lifetime of the chromatography matrix. For example, a sensitive matrix may be cleaned with standard CIP, if a reduced performance is acceptable. The performance of column packed with a chromatography matrix is easily verified using well known methods.

WO 01/27623 discloses a method of regenerating a resin for removing isoagglutinins from blood. The method involves the use of urea, sodium thiosulfate and acetic acid, followed by equilibration.

Brorson et al (Kurt Brorson, Janice Brown, Elizabeth Hamilton, Kathryn E. Stein in Journal of Chromatography A, 989 (2003) 155-163: Identification of protein A media performance attributes that can be monitored as surrogates for retrovirus clearance during extended re-use") describes how Protein A media can be re-used after cleaning with 6M urea or 6 M guanidine hydrochloride, which are known as milder cleaning buffers than sodium hydroxide. It is concluded that column performance was stable even after more than 300 cycles. However, use of urea involves certain drawback. For once, it is a relatively costly chemical at present. Secondly, due to its fertilising effect, it cannot be readily disposed of without taking certain precautions to obey with legislation.

Thus, there is still a need in this field of alternative cleaning protocols for chromatography matrices, especially for use with more labile materials.

Brief description of the present invention

The present invention relates to problems associated with the re-use of separation matrices, preferably chromatography matrices. Illustrative such problems are fouling of packed chromatography matrices and the building up of back pressure during operation. thus, one aspect of the present invention is a process of regenerating a separation matrix. This may be achieved using a protocol comprising at least one reducing regeneration, as defined in the appended claims.

A specific aspect of the invention is a process of regenerating a chromatography matrix which comprises labile ligands and/or support materials.

Other aspects and advantages of the present invention will appear from the detailed description that follows.

Brief description of drawings

Figure 1 shows a comparison of selected chromatograms during lifetime study including reducing regeneration according to the invention.

Figure 2 shows the host cell protein concentration in the elution peaks from an extended cleaning protocol including reducing regeneration according to the invention.

Figure 3 shows the yield (%) versus cycle number from an extended cleaning protocol according to the invention.

Figure 4 shows the peak broadening of elution peaks obtained after reducing regeneration according to the invention (triangles, lower curve) and without wash with reducing agent (filled circles, upper curve).

Figure 5 shows the leakage of Protein A during a control experiment as described in the Experimental part.

Definitions

The term "regeneration" of a chromatography matrix means herein to a process which substantially restores the matrix to its original strength or properties.

The term "chromatography matrix" means herein a stationary phase for use in chromatography, also known as a resin. A chromatography matrix is commonly comprised of a porous or non-porous solid support, to which a plurality of ligands have been coupled, directly or via spacers or extenders.

The term "ligand" is used herein as conventionally used within the field of chromatography, i.e. for a group or a compound, which comprises at least one functional group. The term "alkaline-labile" means herein sensitivity to alkaline concentrations corresponding to pH values in the region of 10-14.

The term "proteinaceous ligands" means herein ligands that comprise proteins and/or protein-like molecules such as peptides.

The term "eluent" means herein a liquid capable of releasing target molecules from a chromatography matrix. The releasing action may e.g. be provided by the pH and/or the conductivity of the eluent.

The term "target molecules" is used herein for any specific molecule or kind of molecule that adsorbs to the chromatography matrix in question, and embraces compounds and cells as well as actual molecules.

The term "break-through capacity" is defined as the amount of target molecules than can be applied to a chromatography matrix, normally packed in a column, before break-through of target molecules in the effluent. The term $Q_{B10\%}$, is commonly used, and refers to the point when the effluent concentration reaches 10% of the initial sample concentration.

Detailed description of the invention

In a first aspect, the present invention relates to a process of regenerating a separation matrix as defined in claim 1.

As the skilled person in this field will easily realise, each added solution, such as eluent, solution comprising the reducing agent, buffers etc are advantageously withdrawn from the matrix before the next one is added. In the most advantageous embodiment of the process, the chromatography matrix is present in a chromatography column, such as an axial or radial chromatography column. In one embodiment, the liquids are added and withdrawn as in batch adsorption chromatography. In an alternative embodiment, the liquids are passed across the column by pumping; by gravity; or by use of a pressure differential.

In an advantageous embodiment, the present process comprises acidic regeneration by contacting the chromatography matrix with an acidic solution at any time after elution but before equilibration of the chromatography matrix. In a specific embodiment, the acidic regeneration is carried out after the reducing regeneration.

As the skilled person in this field will recognize, adding a reducing agent in a chromatographic process may entail the risk of retained reducing agent in the matrix, which could potentially harm or contaminate the target molecule(s). However, by performing at least one of acidic and alkaline regeneration subsequent to the addition of reducing agent, such risk is minimized or even eliminated. Thus, in one embodiment, the acidic and alkaline regenerations are carried out subsequent to the reducing regeneration. In a specific embodiment, the order of steps after elution is reducing regeneration; acidic regeneration; alkaline regeneration; and equilibration.

The present process may be utilised with any kind of chromatography matrix, provided the support and the ligands are capable of withstanding the reducing regeneration. Thus, the process is applicable to regeneration of matrices for ion exchange, such as cation exchange and anion exchange; hydrophobic interaction chromatography (HIC) matrices; immobilised metal affinity chromatography (IMAC) matrices; and affinity matrices, such as with proteinaceous ligands.

However, chromatography matrix comprising bonds or groups that are susceptible to reduction should be avoided. For example, some bonds such as disulfide bonds which will be readily reduced should be avoided. In the process of the invention, the chro-matography matrix comprises proteinaceous ligands devoid of reducible disulfide bonds. The ligands of the chromatography matrix do not comprise any such reducible bonds.

The present invention is especially advantageously used for regenerating a chromatography matrix which is sensible to the conventionally used regeneration protocol using harsh alkaline conditions, commonly using 1M NaOH. Thus, in an advantageous embodiment, the ligands are alkaline-labile. As is well known, due to their composition, proteinaceous ligands are usually sensitive to harsh alkaline conditions, such as 1M NaOH. Thus, in an advantageous embodiment, the proteinaceous ligands comprise Protein A. As is well known, Protein A, which presents a peptidic backbone and no disulfide bonds, is a commonly used protein ligand due to its superior specificity to antibodies. Protein A separation matrices are commercially available, such as the product line Mab-Select™ (GE Healthcare, Uppsala, Sweden).

As the skilled person will readily recognize, the discussion above regarding ligands susceptible to reduction applies equally to the support material. The above-discussed ligands may be coupled to any well known kind of porous or non-porous support, which may be in the form of particles, such as essentially spherical particles, a monolith, filter, membrane, surface, capillaries, etc. In one embodiment, the support is prepared from a native polymer, such as cross-linked carbohydrate material, such as agarose, agar, cellulose, dextran, chitosan, carrageenan, gellan, alginate etc. To obtain high adsorption capacities, the support is preferably porous, and ligands are then coupled to the external surfaces as well as to the pore surfaces. Such native polymer supports are easily prepared according to standard methods, such as inverse suspension gelation (S Hjertén: Biochim Biophys Acta 79(2), 393-398 (1964).

Alternatively, the support is prepared from a synthetic polymer, such as cross-linked synthetic polymers, e.g. styrene or styrene derivatives, divinylbenzene, acrylamides, acrylate esters, methacrylate esters, vinyl esters, vinyl amides etc. Such synthetic polymers are easily produced according to standard methods; see e.g. "Styrene based polymer supports developed by suspension polymerization" (R Arshady: Chimica e L'Industria 70(9), 70-75 (1988)).

In yet an alternative embodiment, the support of the chromatography matrix which is regenerated according to the invention is prepared from an inorganic material, such as glass or silica. In a specific embodiment, the support is comprised of controlled pore glass (CPG) particles.

Immobilising ligands to anyone of the above-discussed supports is also easily performed by the skilled person in this field following well-known methods; see e.g. Immobilized Affinity Ligand Techniques, Hermanson et al, Greg T. Hermanson, A. Krishna Mallia and Paul K. Smith, Academic Press, INC, 1992.

However, chromatography matrices suitable for regeneration according to the present invention are also readily available from commercial sources, such as the Sepharose™ and Source™ series (GE Healthcare Bio-Sciences, Uppsala, Sweden), which include ion exchangers and hydrophobic interaction chromatography matrices. In an advantageous embodiment, the chromatography matrix is MabSelect™ or MabSelect Xtra™ (GE Healthcare Bio-Sciences, Uppsala, Sweden).

In an advantageous embodiment, the adsorption of target molecule(s) is advantageously carried out to a chromatography matrix equilibrated with a buffer. Such buffers are readily available from commercial sources and easily selected by the skilled person in this field depending on the nature of the chromatography matrix and target molecule(s).

In an advantageous embodiment, the washing of the chromatography matrix to which target molecule(s) have been adsorbed is carried out by contacting the chromatography matrix with a buffer. The buffer may be any suitable buffer, such as the same kind used for equilibration.

In an advantageous embodiment, the elution is carried out by a stepwise or continuous pH gradient. Such gradients, and useful methods for providing them e.g. by buffer blending, are well known in this field. The eluent is easily selected by the skilled person in this field depending on the nature of the chromatography matrix and target molecule(s).

The reducing regeneration may be performed using any suitable reducing agent, preferably in the form of a solution, such as DTE, DTT, mercaptoethanol, L-cysteine, and thioglycerol, which are all readily commercially available. In one embodiment, the reducing agent comprises one or more thiols. In a specific embodiment, the reducing agent comprises thioglycerol. The optimal pH for reducing regeneration will be dependent on the reducing agent selected, and will commonly be in a range of 8-8.5. Thus, in one embodiment, the reducing regeneration is carried out at alkaline pH.

The acidic regeneration may be performed using any suitable acid, such as acetic acid. In one embodiment of the present process, the acidic regeneration is carried out at pH below 3. As the skilled person in this field will recognize, to obtain the most advantageous acidic regeneration, some processes may require certain conductivity. Thus, in one embodiment, the acidic regeneration is carried out with a solution comprising salt. Illustrative salts are e.g. sodium sulphate ($Na_2SO_4$) and sodium chloride (NaCl).

The alkaline regeneration may be performed using any suitable alkaline agent, such as sodium hydroxide of a suitable concentration. In one embodiment of the present process, the alkaline regeneration is carried out at pH in the range of 10-14, such as 11-13. In one embodiment, the pH is 11-12. In another embodiment, the pH is 12-13. As the skilled person in this field will recognize, to obtain the most advantageous alkaline regeneration, some processes may require certain conductivity. Thus, in one embodiment, the alkaline regeneration is carried out with a solution comprising salt. Illustrative salts are e.g. as exemplified above in the context of the acidic regeneration.

Detailed description of the drawings

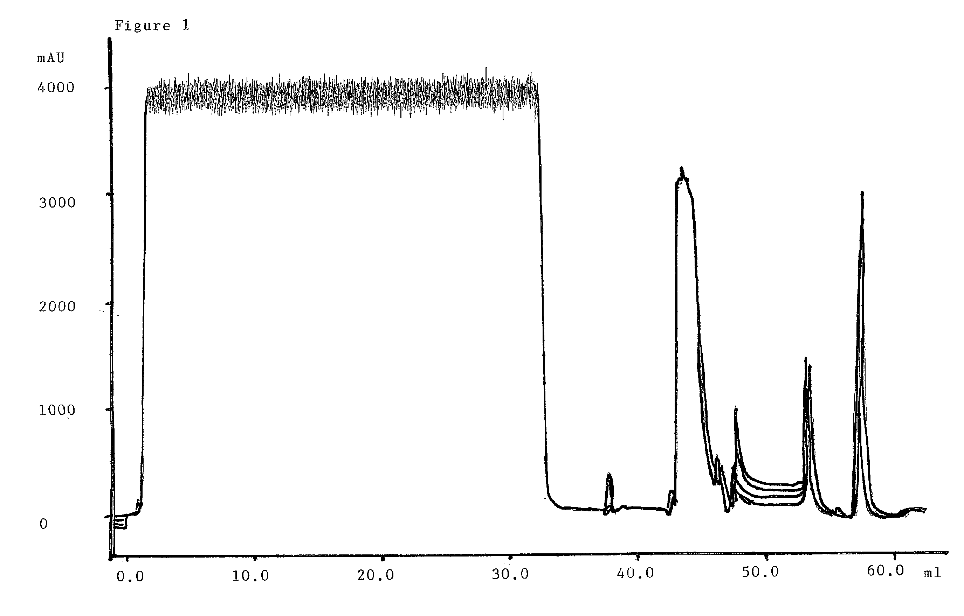

Figure 1 shows a comparison of selected chromatograms during lifetime study including reducing regeneration according to the invention, as described in example 1 below. More specifically, cycle 6 (shown in blue), cycle 21 (shown in red), cycle 40 (shown in brown) and cycle 60 (shown in green) illustrates how little the separation matrix is affected by repeated cleaning protocols according to the invention.

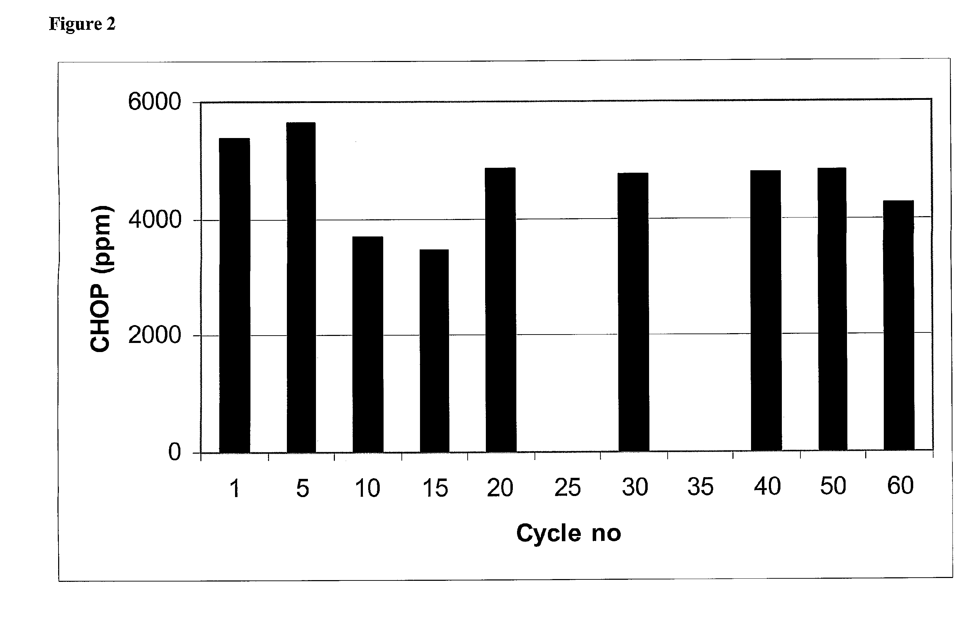

Figure 2 shows the host cell protein concentration in the elution peaks from an extended cleaning protocol including reducing regeneration according to the invention. The reducing agent is 1-thioglycerol, and the concentration of host cell protein (CHOP) is in ppm.

As appears from Figure 2, the concentration of host cell protein is essentially unchanged, which means that the separation matrix is still capable of removing undesired components even after a large number of regeneration cycles.

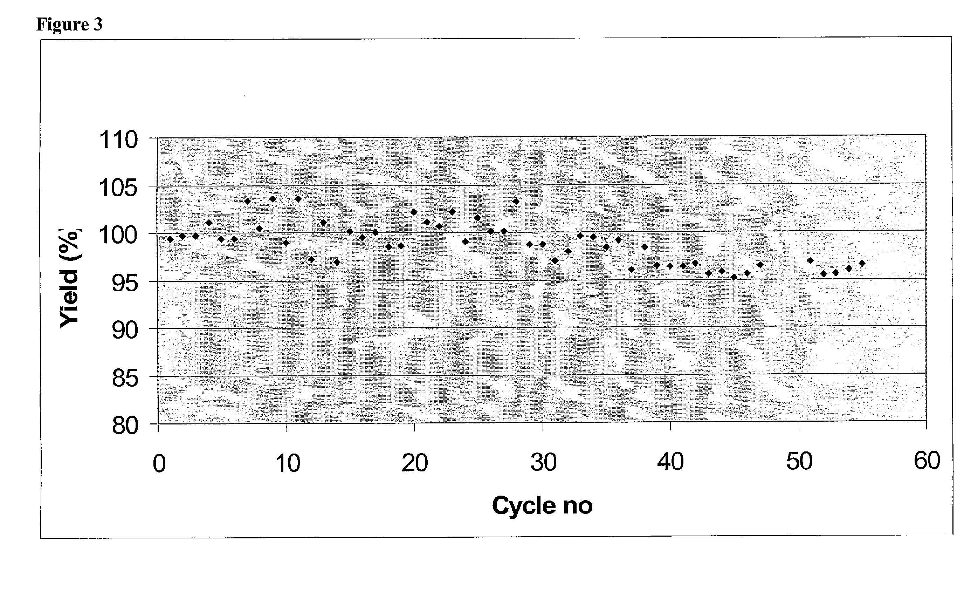

Figure 3 shows the yield (%) versus cycle number from an extended cleaning protocol according to the invention, wherein the reducing agent is 1-thioglycerol. As expected from a well functioning cleaning protocol, the yield remains substantially unaltered.

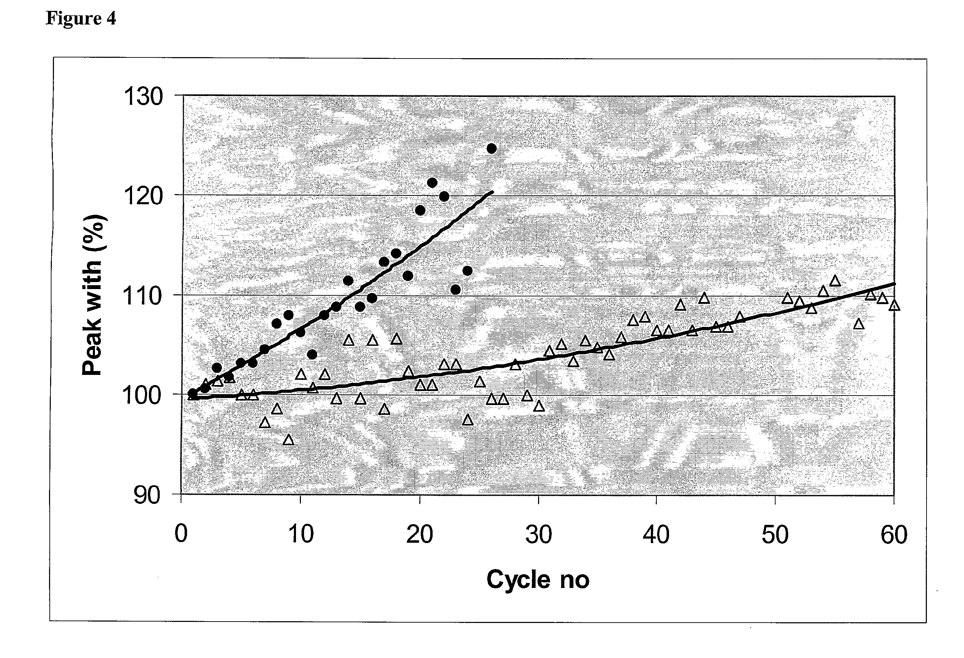

Figure 4 shows the peak broadening of elution peaks obtained after reducing regeneration according to the invention (triangles, lower curve) as described in example 3 below; and a conventional cleaning protocol without wash with reducing agent (filled circles, upper curve). As appears from Figure 4, the conventional cleaning protocol leads to peak broadening much sooner than the protocol according to the invention.

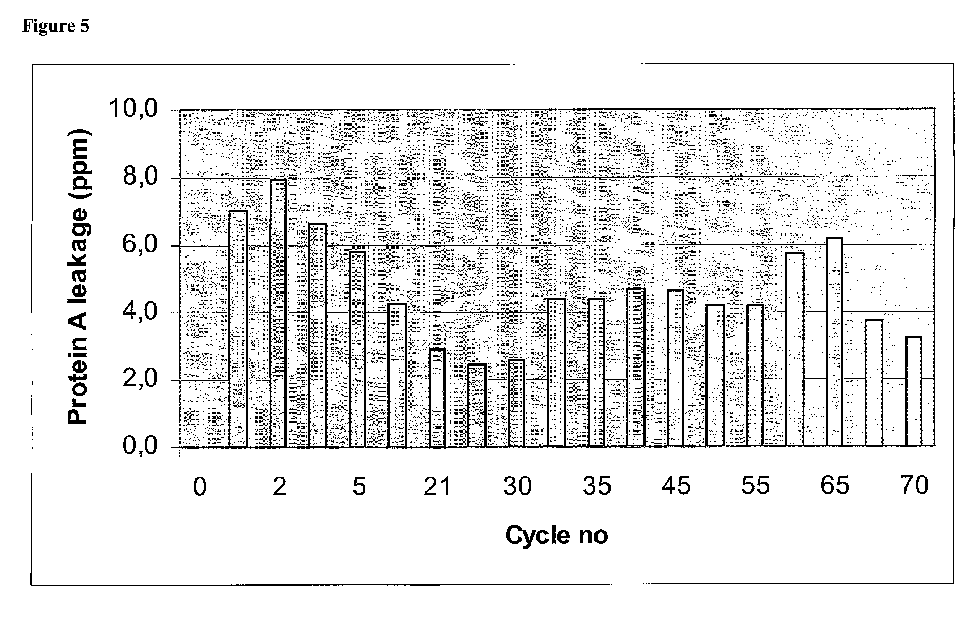

Figure 5 shows the leakage of Protein A from an affinity matrix during a control experiment as described in the Experimental part below. As appears from Figure 5, the leakage does not present any substantial increase even after a large number of cycles, which means that the binding of Protein A ligands to the separation matrix is not affected to any substantial degree by the cleaning protocol according to the invention.

EXPERIMENTAL PART

The following examples are provided for illustrative purposes only, and should not in any way be construed as limiting the scope of the invention as defined by the appended claims.

**Materials / Investigated units**

Instruments

Chromatography system ÄKTA™ Explorer 10, HJ E-10, with UNICORN v. 4.11
Spectrophotometer Ultrospec 3000pro, no 839

Columns

HR 5/5, GE Healthcare Bio-Sciences

Chemicals/other

| | |
|---|---|
| Acetic acid, Merck, cat. no. 1.00063, p.a. | Benzyl alcohol, Merck, cat. no. 1.09626, p.a. |
| HCl, Merck, cat. no. 1.00317, p.a. | Na-citrate, Merck, cat. no. 1.06448, p.a. |
| NaCl, Merck, cat. no. 1.06404, p.a. | $NaN_3$, BDH, cat. no. 103692K, Merck cat. no. 6688 |
| NaOH, Merck, cat. no. 1.06469, p.a. | $Na_2SO_4$, Merck, cat. no. 1.06649, p.a. |
| Tris, Merck, cat. no. 1.08382, p.a. | |

1-thioglycerol 90, from GE Healthcare Bio-Sciences (raw material supply) 30-2507-00

KCl, Merck, cat no 4936.1000

EDTA, anhydrous, SIGMA

Water: MilliQ

Filters: 1.2 $\mu$m, 0.45 $\mu$m, 0.22 $\mu$m, Millipore

Resins

MabSelecXtra™

Buffers

| | |
|---|---|
| Equilibration buffer: | 25 mM Tris, 0.15 M NaCl, pH 7.4 |
| Elution buffer: | 100 mM acetic acid, pH 3.6 |
| Neutralisation buffer: | 1 M Tris pH 9.0 (collected fractions in test tubes) |
| Acidic regeneration buffer. | 1 M acetic acid, 50 mM $Na_2SO_4$ |
| Wash (reducing agent) | 100 mM 1-thioglycerol, 25 mM TRIS, 0.15 M NaCl, 25 mM KCl, 1 mM EDTA pH 8.5 |
| Alkaline regeneration solution: | 50 mM NaOH, 0.5 M $Na_2SO_4$ |
| Storage buffer: | 2 % Benzyl alcohol, 50 mM Na-citrate, pH 5.0 |

Samples

In this example, feed containing fusion protein expressed in Chinese hamster ovary (CHO) cells was used. The expression of protein was carried out following well known methods. The feed contained 0.48 mg/mL of the fusion protein.

Polyclonal human IgG, Gammanorm, was obtained from Octapharma AB.

Analyses

The protein A and host cell protein content (CHOP) was determined in the eluate pools of selected cycles by ELISA.

Example 1: Column packing

HR 5/5 column were filled with 4 M NaCl. A packing tube (HR 16) was connected, and was filled with 20% gel slurry in ∼ 0.2 M NaCl. Packing was then performed in Milli Q water at 3 ml/min for 3 min. The packing tube was then disconnected, and a top adaptor was lowered towards the gel surface. After additional packing at 3 ml/min, the adaptor was adjusted 1 mm into the bed. Packing was then continued at 1 ml/min for 20 minutes. Packing performance (i.e. plate number and asymmetry) was evaluated by injection of 100 $\mu$l 2% acetone at a flow rate of 0.35 mL/min. The acceptance criteria for the column packing were an asymmetry between 0.8 - 1.33 and number of theoretical plates > 2000 N/m.

Example 2: Purification protocol according to the invention

The method is summarised in table 1 below. The buffer compositions are found in section Materials/Investigated units above. The UV was detected at 280 nm.

Table 1: A cleaning protocol according to the invention

| Step | Amount | Flow (cm/h) | Buffer | Comment |
|---|---|---|---|---|
| Equilibration | 6 CV | 300 | Equilibration | |
| Load | 15-18 mg/ml* | 206 | CHO supernatant | 6 min residence time, load to 15-18 mg/mL media |
| Wash | 6 CV | 300 | Equilibration | |
| Wash | 5 CV | 300 | 25 mM TRIS pH 8.0 | |
| Elution | varied | 300 | Elution | Start/stop collect at 300 mAU |
| Reducing reg | 6 CV** | 300 | Reducing buffer | 100 mM 1-thioglycerol*** |
| Acid regener | 3 CV | 206 | Strip | |
| Wash | 1 CV | 206 | Equilibration | |
| Basic regene | 3 CV | 103 | CIP solution | 12 min residence time |
| Wash | 0.5 CV | 103 | Equilibration | 12 min residence time, performed every two cycles |
| Storage | 3 CV | 103 | Storage | 12 min residence time, performed every two cycles |

*The sample load was decreased to 15 mg/ml, i.e. 82% of $Q_{B,10\%}$. In addition a control experiment was performed with human IgG in equilibration buffer as described below.
** A control experiment was performed with 0 CV, i.e. without the reducing regeneration according to the invention.
*** New 1-thioglycerol solution was prepared every day.

Example 3: Investigation of eluate

Neutralisation of eluate and absorbance measurement

The eluate was collected in test tubes to which 100 $\mu$l of neutralisation buffer had been added. The eluate was diluted (1:20) in equilibration buffer. The concentration of the sample solution was determined at 280 nm in a spectrophotometer and calculated according to Lambert Beer's law. The average value of the absorbance was used for concentration determination.

Protein A leakage

Neutralized eluate was measured by ELISA as described in Steindl F and et al. A simple method to quantify staphylococcal protein A in the presence of human or animal IgG in various samples. J Immunol Meth (2000) 235, 61-9.

Frontal analysis with pure fusion protein

Frontal analysis was performed according to well known methods. The breakthrough capacity ($Q_{B10\%}$) was calculated according to

$$Q_{B10\%} = (V_{10\%} - Vo)Co/Vc$$

were $V_{10\%}$ = applied sample volume at 10% breakthrough, Vo = void volume, Co = sample concentration (mg/ml) and Vc = geometric total volume (ml).

Results example 3

A lifetime study using regeneration with 1-thioglycerol was performed for 60 cycles. A selection of chromatograms is presented in figure 1. As can be seen, the chromatograms are quite similar, even though the volume of the elution peaks gradually increased. The total peak broadening was about 7 % for 40 cycles and 10% for 60 cycles (figure 4, triangles). This is a significant improvement compared to the standard protocol (figure 4, filled circles).
Results from measurements of host cell protein levels (CHOP) in the elution peaks are shown in fig. 2. No significant changes could be observed in the CHOP level throughout the study.

The yield was relatively stable (>95%) for 60 cycles. Slightly decreased values were obtained after cycle 37, but no specific trend could be observed (figure 3). The lower values occurred directly after change to a new bottle of feedstock, and are probably caused by variation in protein concentration. As a control, frontal analysis with pure fusion protein was performed after 55 cycles. The result revealed that the breakthrough capacity ($Q_{B,10\%}$) was unaltered compared to the initial capacity (i.e. 18 mg/ml **) (results not shown).

The column performance in this study, using HR 5/5 columns, was maintained after 55 cycles (table 2).

Table 3: Column performance before and after 55 purification cycles.

| Packing performance (i.e. plate number and asymmetry) was evaluated by injection of 100 µl 2% acetone at a flow rate of 0.2 mL/min. | | |
|---|---|---|
| **Column** | **Asymmetry** | **Number of theoretical plates** |
| New | 1,11 | 2367 |
| After 55 cycles | 1,19 | 2668 |

Example 4: Comparative examples

Control experiment 1: With and without reducing regeneration

A control experiment was performed for 26 cycles by use of the same method as above, but without wash with reducing agent (i.e. without reducing regeneration). The result clearly shows that broadening of the elution peaks is much more serious and accelerated (figure 4). Thus, 10% peak broadening is obtained already after 15 cycles (compared to 60 cycles of 1-thioglycerol). The total peak broadening after 26 cycles was about 20%.

Control experiment 2: Protein A leakage

70 cycles was performed with the same method as above, but human IgG (28 mg/ml) was loaded to the column instead of fusion protein-containing feed. The Protein A leakage was very low (i.e. ≤8 ppm), and no increase in leakage levels could be observed (figure 5, table 3).

Table 3. Control experiment 2 - Protein A leakage

| Protein A leakage | | |
|---|---|---|
| Cycle no | ng/ml | ppm |
| 0 | 0 | 0,0 |
| 1 | 78,9 | 7,1 |
| 2 | 85,15 | 8,0 |
| 4 | 98,65 | 6,7 |
| 5 | 77 | 5,8 |
| 12 | 61,4 | 4,2 |
| 21 | 31,9 | 2,9 |
| 25 | 32,05 | 2,5 |
| 30 | 28,35 | 2,6 |
| 35 | 39,95 | 4,4 |
| 35 | 39,55 | 4,4 |
| 40 | 40,1 | 4,7 |
| 45 | 38,15 | 4,7 |
| 50 | 37,9 | 4,2 |
| 55 | 38,1 | 4,2 |
| 60 | 51,7 | 5,7 |
| 65 | 58,7 | 6,2 |
| 69 | 34,8 | 3,7 |
| 70 | 29,2 | 3,2 |

**Claims**

1. A process of regenerating a separation matrix comprising

    (a) providing a separation matrix from which an adsorbed sample has been eluted;
    (b) reducing regeneration by contacting said matrix with a reducing agent which comprises one or more thiols;
    (c) alkaline regeneration by contacting the matrix with an alkaline solution;
    (d) equilibration of the matrix;

    wherein the regeneration steps are carried out in any order of sequence, wherein the alkaline regeneration is carried out at pH in the range of 10-13, and wherein the separation matrix comprises proteinaceous ligands, which are coupled to a support and which are devoid of disulfide bonds.

2. A process according to claim 1, further comprising acidic regeneration by contacting the matrix with an acidic solution at any time after elution but before equilibration of the matrix.

3. A process according to claim 2, wherein the acidic regeneration is carried out after the reducing regeneration.

4. A process according to any one of claims 2 or 3 , wherein the order of steps after elution is reducing regeneration; acidic regeneration; alkaline regeneration; and equilibration.

5. A process according to any one of the preceding claims, wherein steps (a)-(c) are carried out 1-5 times, such as 2-5 times.

6. A process according to any one of the preceding claims, wherein the proteinaceous ligands comprise Protein A.

7. A process according to any one of the preceding claims, wherein the reducing regeneration is carried out at alkaline pH.

8. A process according to any one of the preceding claims, wherein the reducing agent comprises thioglycerol.

9. A process according to any one of claims 2 or 3, wherein the acidic regeneration is carried out at pH below 3.

10. A process according to any one of claims 2 or 3, wherein the acidic regeneration is carried out with a solution comprising salt.

11. A process according to any one of the preceding claims, wherein the alkaline regeneration is carried out with a solution comprising salt.

**Patentansprüche**

1. Verfahren zum Regenerieren einer Trennmatrix umfassend

    (a) Bereitstellen einer Trennmatrix wovon eine absorbierte Probe eluiert wurde;
    (b) Reduzierende Regeneration durch In-Kontakt-bringen der Matrix mit einem Reduktionsmittel welches ein oder mehrere Thiole umfasst;
    (c) Alkalische Regeneration durch In-Kontakt-bringen der Matrix mit einer alkalischen Lösung;
    (d) Equilibrieren der Matrix;

    wobei die Regenerationsschritte in beliebiger Reihenfolge durchgeführt werden,
    wobei die alkalische Regeneration bei einem pH-Wert im Bereich von 10 bis 13 durchgeführt wird und wobei die Matrix proteinartige Liganden umfasst, die an einen Träger gekoppelt sind und keine Disulfidbindungen enthalten.

2. Verfahren nach Anspruch 1 weiterhin umfassend eine saure Regeneration durch In-Kontakt-bringen der Matrix mit einer sauren Lösung zu einer beliebigen Zeit nach der Elution jedoch vor dem Equilibrieren der Matrix.

3. Verfahren nach Anspruch 2, wobei die saure Regeneration nach der reduzierenden Regeneration durchgeführt wird.

**4.** Verfahren nach einem der Ansprüche 2 oder 3, wobei die Reihenfolge der Schritte nach der Elution reduzierende Regeneration, saure Regeneration, alkalische Regeneration und Equilibrierung ist.

**5.** Verfahren nach einem der vorherigen Ansprüche, wobei die Schritte (a) bis (c) ein bis fünfmal, z.B. zwei bis fünfmal, durchgeführt werden.

**6.** Verfahren nach einem der vorherigen Ansprüche, wobei die proteinartigen Liganden Protein A umfassen.

**7.** Verfahren nach einem der vorherigen Ansprüche, wobei die reduzierende Regeneration bei einem alkalischen pH-Wert durchgeführt wird.

**8.** Verfahren nach einem der vorherigen Ansprüche, wobei das Reduktionsmittel Thioglycerin umfasst.

**9.** Verfahren nach einem der Ansprüche 2 oder 3, wobei die saure Regeneration bei einem pH-Wert von weniger als 3 durchgeführt wird.

**10.** Verfahren nach einem der Ansprüche 2 oder 3, wobei die saure Regeneration mit einer Lösung durchgeführt wird, die Salz umfasst.

**11.** Verfahren nach einem der vorherigen Ansprüche, wobei die alkalische Regeneration mit einer Lösung durchgeführt wird, die Salz umfasst.

**Revendications**

**1.** Procédé de régénération d'une matrice de séparation, comprenant les étapes consistant à :

   (a) fournir une matrice de séparation dont un échantillon adsorbé a été élué ;
   (b) procéder à une régénération réductrice en mettant en contact ladite matrice avec un agent réducteur qui comprend un ou plusieurs thiols ;
   (c) procéder à une régénération alcaline en mettant en contact la matrice avec une solution alcaline ;
   (d) équilibrer la matrice ;

   dans lequel les étapes de régénération sont effectuées dans un ordre de séquence quelconque,
   dans lequel la régénération alcaline est effectuée à pH dans la plage de 10 à 13, et
   dans lequel la matrice de séparation comprend des ligands protéiniques qui sont couplés à un support et sont exempts de liaisons disulfure.

**2.** Procédé selon la revendication 1, comprenant en outre une régénération acide par mise en contact de la matrice avec une solution acide à tout moment après l'élution, mais avant l'équilibrage de la matrice.

**3.** Procédé selon la revendication 2, dans lequel la régénération acide est effectuée après la régénération réductrice.

**4.** Procédé selon l'une quelconque des revendications 2 ou 3, dans lequel l'ordre des étapes après l'élution est la régénération réductrice ; la régénération acide ; la régénération alcaline ; et l'équilibrage.

**5.** Procédé selon l'une quelconque des revendications précédentes, dans lequel les étapes (a) à (c) sont effectuées 1 à 5 fois, notamment 2 à 5 fois.

**6.** Procédé selon l'une quelconque des revendications précédentes, dans lequel les ligands protéiniques comprennent de la Protéine A.

**7.** Procédé selon l'une quelconque des revendications précédentes, dans lequel la régénération réductrice est effectuée à pH alcalin.

**8.** Procédé selon l'une quelconque des revendications précédentes, dans lequel l'agent réducteur comprend du thioglycérol.

9. Procédé selon l'une quelconque des revendications 2 ou 3, dans lequel la régénération acide est effectuée à un pH en dessous de 3.

10. Procédé selon l'une quelconque des revendications 2 ou 3, dans lequel la régénération acide est effectuée avec une solution comprenant du sel.

11. Procédé selon l'une quelconque des revendications précédentes, dans lequel la régénération alcaline est effectuée avec une solution comprenant du sel.

Figure 1

Figure 2

CHOP (ppm)

Cycle no

Figure 3

Figure 4

Figure 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0127623 A

**Non-patent literature cited in the description**

- **KURT BRORSON ; JANICE BROWN ; ELIZABETH HAMILTON ; KATHRYN E. STEIN.** Identification of protein A media performance attributes that can be monitored as surrogates for retrovirus clearance during extended re-use. *Journal of Chromatography A,* 2003, vol. 989, 155-163
- **S HJERTÉN.** *Biochim Biophys Acta,* 1964, vol. 79 (2), 393-398
- **R ARSHADY.** Styrene based polymer supports developed by suspension polymerization. *Chimica e L'Industria,* 1988, vol. 70 (9), 70-75
- **HERMANSON et al.** Immobilized Affinity Ligand Techniques. Academic Press, INC, 1992
- **STEINDL F et al.** A simple method to quantify staphylococcal protein A in the presence of human or animal IgG in various samples. *J Immunol Meth,* 2000, vol. 235, 61-9